# EUROPEAN PATENT APPLICATION

(11) **EP 1 604 677 A1**
(43) Date of publication of application: **14.12.2005**
(21) Application number: 05105050.8
(22) Date of filing: 09.06.2005
(51) Int. Cl.: A61K 35/78, A61K 38/43, A23L 2/02

(54) **Composition having antioxidant, anti-inflammatory, and immunostimulant actions**

(30) Priority: 10.06.2004 IT TO20040390
(71) Applicant: ROEDER 1956 FARMACEUTICI S.p.A., 10126 Torino (IT)
(72) Inventor: Villa, Claudio, I-10131, TORINO (IT)
(74) Representative: Gerbino, Angelo

(57) **Abstract**

The composition, in particular for pharmaceutical, dietetic and alimentary use, comprises extracts or juices of papaya, aloe and noni. The extracts and juices of aloe and papaya have a proteolytic activity not greater than 5 mcu/g. Advantageously, the extracts or juices of papaya, aloe and noni are present in a ratio by weight lying between 1000:1000:1 and 1:1:100 and preferably between 100:100:1 and 1:1:10.

## Description

The present invention relates to a composition, in particular for pharmaceutical use, having antioxidant, anti-inflammatory and immuno-stimulant action, comprising extracts or juices of papaya (Carica papaya Linn.), aloe (Aloe vera Tourn.) and noni (Morinda citrifolia).

The characteristics of each of these three plants are widely known in the scientific literature, as well as the properties possessed by the various types of their extracts.

Papaya and aloe in particular have been known for a long time and utilised for the pharmacological properties of several substances contained in them. The fruit of papaya, in fact, contains an active substance of enzymatic nature, papaina, which has a proteolytic action.

This substance finds wide use in medicine. It has been, and still is, utilised in pharmaceutical compositions aimed at improving the digestive processes. Single component formulations based on papaina have been utilised in therapies for the treatment of inflammation processes, in particular of soft tissues.

For local use, papaina, in a suitable vehicle, has been utilised in the treatment of chronic lesions of the skin in secretion phase.

Preparations containing high doses of the papaina enzyme are reserved for specialist medical use in that this substance can give rise to hypersensitivity reactions and, if badly utilised, to lesions of the stomach walls and intestine. No contra-indications exist, on the other hand, against the use of the product as a food, that is to say in the consumption of the correctly ripened fruit. In this case, rather, the benefits related to the consumption of a food of high nutritive quality are united with those relating to the presence of physiologically active substances able to promote the digestive process.

Finally, the preparations based on papaya obtained by fermentation processes produced by yeasts characteristic of the same fruit (Pennisetum purpureum Schum., Sechium edule Swartz) have recently been used. The products deriving from these processes acquire, in comparison with the starting product, increased neutraceutic properties. The natural fermentation of papaya juices significantly increases the antioxidant properties of the fruit, probably through the formation of new molecules, reduces the presence of the proteolytic papaina enzymes and chimo-papaina, significantly modifies the protein and carbohydrate ratio to the benefit of these latter. Among the carbohydrates present in the fermented products are found numerous new classes of oligosaccharides of different degrees of polymerisation, structurally comparable to the base structure of beta 1-3 glucan.

The scientific literature contains much documentation of the fact that oligosaccharides of this type possess immuno-stimulant properties in different amounts.

Aloe also possesses this possibility of dual use. Alongside a more properly medicinal use, related to the presence of pharmacologically active substances localised in the cortical parts of the leaf of this plant there is the food use characterised by the use of juices obtained by pressing the internal part of the leaf.

From the cortical part of the leaf is derived a lattice containing aloin, an anthroquinonic pentoxide or a mixture of variable pentoxides in the composition depending on the provenance of the starting plant species. Aloin, conventionally called barbaloin when derived from aloe vera barbadiensis, has in the past been widely utilised for its laxative and cathartic properties. Such properties are due to the action of active anthroquinonic derivatives which form at the intestinal level by hydrolysis of the aloin. The laxative action of the aloin manifests itself after eight hours from consumption. The use of aloin has a smaller following than in the past in view of the greater safety and tolerability of other preparations based on anthroquinones currently in use.

Among the consolidated therapeutic uses of aloe, widely supported by scientific evidence, primary importance is given to that relating to the topical use of juices obtained from the medullary part of the leaves, rich in mucillagins, in the treatment of ulcerative lesions and dermatitis of the skin. These preparations, which manifest interesting antiseptic, anti-inflammatory and revitalising properties, contain active substances of muco-polysaccharidic nature; they do not, on the other hand, contain anthroquinonic derivatives. These characteristics are essential for their dietetic/neutraceutic use. In particular juices and extracts containing less than 0.5% anthroquinones expressed as aloin are acceptable, whilst the active fraction constituted by polysaccharides, preferably expressed as acemannan, must be greater than 1% even in un-concentrated juice obtained by pressing.

Acemannans have immuno-stimulant properties related to their capacity, once absorbed at the intestinal level, to stimulate, at the level of the thymus and beta pancreatic cells, formation of T-lymphocites and macrophages. In the active polysaccharidic fraction are also comprised glucomannans which, together with substances of other nature - saponine, enzymes, anthroquinonic pentoxides - contribute to the protective, anti-inflammatory and healing action on skin and mucus.

Noni is a plant originating in Hawaii and the islands of the South Pacific and is today substantially diffused throughout the sub-tropical zones of the austral hemisphere. Unlike aloe and papaya, it is completely unknown to Western medicine both official and traditional. On the contrary, in the areas of diffusion, and particularly in Polynesia, there is a consolidated ethno-pharmacological use of every part of the plant with a wide spectrum of indications. Noni is, in effect, a rich source of substances having antioxidant action such as Vitamin C, carotenoids and selenium.

Noni and its extracts have, moreover, a significant content of protein and amino acids. This also partly explains the food value in a strict sense of the fruits and the leaves of this vegetable which for a long time have been utilised by the populations of Polynesia for the production of traditional dishes. All the spectrum of the amino acids is contained in noni and this makes it an extremely complete source of nutrition. Numerous studies are in progress for evaluating the possibilities of the use of noni in therapy to confirm the ethno-pharmacological uses which are of wide spectrum.

In fact, noni has been attributed with antibacterial, antiviral, antifungal, anthelmintic, analgesic, hypertensive, anti-inflammatory and immuno-stimulant properties for which definitive confirmation by official science is still needed. In particular, one molecule - the damnacantal - isolated from the roots of noni, has been shown experimentally to have interesting anti-tumoral properties.

In the juice of noni, obtained by pressing the fruit, have been found immuno-modulant polysaccharides for which a promising anti-tumoral action has been shown experimentally. Studies performed up to now have sought to give an explanation to such a wide spectrum of pharmacological action by identifying in it the presence of an alkaloid - xeronine - and in particular of significant quantities of the system constituted by its precursor - proxeronine - and by the activator enzyme - proxeronase.

Xeronine performs an important role in various metabolic processes allowing, at the cellular level, a facilitated access to molecules of a proteic nature (enzymes, co-enzymes and other factors of significant biological relevance) in their sites of action. Xeronine is an extremely active, but at the same time unstable, molecule. Noni on the other hand contains significant quantities of its precursor and of the enzyme able to transform it into the active form. The quantities of proxeronine and proxeronase which pass unaltered through the gastric barrier modulate the intestinal release of xeronine, which in turn modulates the absorption of nutrients and biologically active substances before being inactivated by the pancreatic enzymes.

The present invention is therefore based on the idea of associating an extract or juice of noni with extracts or juices of aloe and papaya for the purpose of increasing the absorption by the organism of the phytocomplexes responsible for the biological actions of these plants.

In this way, the action of noni is complementary to that of aloe and papaya and contains the presuppositions for a strengthening synergy of the anti-inflammatory, immuno-stimulant and antioxidant properties of the extracts.

The extracts and juices of aloe and papaya utilised in association with those of noni must have, as essential requisites, a proteolytic activity not greater than 5 mcu/g which could cause inactivation of the quantities of proxeronine and proxeronase introduced, and therefore of the synergistic effect.

The composition of the present invention is active in the regulation of organic and cellular metabolic functionality which is modified or altered in the physiological process of ageing, and is usable in particular to maintain or improve the efficiency of the antioxidant system and of the immune system by encouraging at the same time the endocrine functionality by means of a neutraceutic action of physiological type not related to therapeutic actions of substitutive type.

The composition of the present invention is moreover usable in the alimentary field, for example for common foods, complementary foods and integratory foods.

In order to encourage the development of the synergistic activity mentioned above, the papaya extracts utilised are preferably subjected to fermentation processes acting substantially to reduce, if not to cancel out entirely, the concentration of the enzymatic fractions having proteolytic activity, but able to promote the presence of the fractions having immuno-stimulant and antioxidant action. It is however possible to utilise for these purposes treatment processes other than fermentation processes on the papaya extracts.

Still preferably, the papaya extract utilised is produced starting from grated fruit coming from a biological cultivation. This fruit is preferably collected before definitive ripening, but fruits in other stages of ripening can also be utilised. As well as pulp, and together with it, can be utilised other parts of the fruit such as the seeds and the skin, in this case eliminating the undesirable components.

The substrate thus obtained and typically added of glucose or other fermentable substances (simple or complex carbohydrates such as sucrose, fructose, lactose, maltodextrin) in the presence of yeasts existing on the fruit (Pennisetum purpureum Schum., Sechium edule Swartz. or the like) is subjected to a natural fermentation process which can be prolonged for up to six consecutive months in controlled conditions until reaching the required composition described above.

Advantageously the extracts or juices of papaya, aloe and noni are present in the composition of the invention in quantities corresponding to a ratio by weight lying between 1000:1000:1 and 1:1:100 and more preferably between 100:100:1 and 1:1:10. The ratio 1:1:1 is greatly preferred.

The composition of the invention can moreover include any pharmaceutically, dietetically and neutraceutically acceptable eccipient and possible other active principles.

In particular, the composition of the invention can contain additives chosen from the group consisting of vitamins, mineral salts, amino acids, vegetable extracts and lactic ferments, either live or inactive, as well as biologically active substances such as antioxidants.

Such antioxidants can for example be inorganic antioxidants, in particular selenium, antioxidants of vitaminic nature, in particular carotenes and their derivatives, as well as sulphurated organic derivates such as methyl sulphonyl methane (MSM), sulphurated amino acids such as cistine, cistein and methionin and their derivates, such as acetyl cistein. Specific examples of further usable additives are vegetable extracts such as Panax ginseng and Eleutherococcus senticosus, and totally or partially live or inactive pre-biotic micro-organisms included among those forming part of the bacterial flor living in the human intestine, such as Lactobacillus acidophilus.

The composition of the invention can be administered in any conventional manner, for example by oral, topical or transdermal means, and with doses - referred to an average individual of 70 kg weight, of at least 0.1 mg/day of papaya extract (200:1), 0.1 mg/day of aloe extract (200:1) and 0.01 mg/day of extract of noni (titrated to 15% in polysaccharides) and preferably 200 mg/day of papaya extract (200:1), 50 mg/day of aloe extract (200:1) and 100 mg/day of noni extract (titrated to 15% in polysaccharides).

Further advantages and characteristics of the present invention will become evident from the following examples of formulations provided by way of non-limitative examples.

### EXAMPLE 1 - Drink based on juices and extracts

| | |
|---|---|
| Juice of papaya fruit flesh | 12.3% |
| Aloe leaf gel, dry extract concentrated to 200:1 | 0.23% |
| Noni fruit, dry extract titrated to 15% in polysaccharides | 0.1% |
| Eccipient: water, fructose, tartaric acid, potassium sorbate, sodium benzoate, flavourings | q.s. to 100% |

### EXAMPLE 2 - Capsules based on extracts

| | |
|---|---|
| Fermented papaya fruit pulp, dry extract | 200 mg |
| Noni fruit, dry extract titrated to 15% in polysaccharides | 100 mg |
| Aloe leaf gel, dry extract 200:1 | 50 mg |
| Eccipients: alimentary gelatine, maltodextrine, magnesium stearate, micronised silica, titanium dioxide. | |

### EXAMPLE 3 - Dermatological cream based on juices and extracts

| | |
|---|---|
| Aloe leaf pulp gel | 40% |
| Papaya fruit pulp dry extract | 0.5% |
| Noni fruit, dry extract titrated to 15% in polysaccharides | 0.5% |
| Eccipients | q.s. to 100% |

Naturally, the principle of the invention remaining the same, the details of realisation and the embodiments can be widely varied with respect to what has been described purely by way of example, without by this departing from its ambit. In particular, such embodiments may include applications of the composition of the invention in other fields such as, for example, dietary and cosmetic.

## Claims

1. A composition, in particular for pharmaceutical, dietetic and alimentary use, comprising extracts or juices of papaya, aloe and noni, the said extracts and juices of aloe and papaya having a proteolytic activity not greater than 5 mcu/g.

2. A composition according to Claim 1, in which the said extracts or juices of papaya, aloe and noni are present in a ratio by weight lying between 1000:1000:1 and 1:1:100 and preferably between 100:100:1 and 1:1:10.

3. A composition according to Claim 2, in which the said extracts or juices of papaya, aloe and noni are present in a ratio by weight of about 1:1:1.

4. A composition according to any preceding Claim, further including one or more additives chosen from the group consisting of vitamins, mineral salts, amino acids, vegetable extracts and lactic ferments, whether live or inactivated.

5. A composition according to any preceding Claim, further containing biologically active substances such as antioxidants.

6. A composition according to Claim 5, containing inorganic antioxidants, in particular selenium.

7. A composition according to Claim 5, containing antioxidant molecules of vitaminic nature, in particular carotene and their derivatives.

8. A composition according to any preceding Claim, containing sulphurated organic derivatives such as methyl sulphonyl methane (MSM), sulphurated amino acids, such as cistine, cistein and methionin and their derivatives such as acetyl cistein.

9. A composition according to any preceding Claim, containing vegetable extracts such as Panax ginseng and Eleutherococcus senticosus.

10. A composition according to any preceding Claim, containing totally or partially live or inactivated micro-organisms including those forming part of the habitual bacterial flor of the human intestine, such as Lactobacillus acidophilus.

11. A composition according to any preceding Claim, further including pharmaceutical and dietetically acceptable eccepients and/or other active principles.

12. A composition according to any preceding Claim, in a form administrable orally, topically or transdermically.

13. Use of a composition according to any preceding Claim for the production of a medicament for the treatment/prevention of alterations in the immune system.

14. Use of a composition according to any preceding Claim from 1 to 12 for the production of a medicament for the treatment of inflammation associated with alterations of the immune system.
